(19) Europäisches Patentamt
European Patent Office
Office européen des brevets

(11) Veröffentlichungsnummer: **0 483 615 A2**

(12) # EUROPÄISCHE PATENTANMELDUNG

(21) Anmeldenummer: **91117800.2**

(22) Anmeldetag: **18.10.91**

(51) Int. Cl.[5]: **C07D 249/08**, C07D 233/58, C07D 233/60, C07D 409/06, C07D 401/06, C07D 405/06, A01N 43/653, A01N 43/50

(30) Priorität: **29.10.90 DE 4034352**

(43) Veröffentlichungstag der Anmeldung:
**06.05.92 Patentblatt 92/19**

(84) Benannte Vertragsstaaten:
**AT BE CH DE DK ES FR GB GR IT LI NL SE**

(71) Anmelder: **BASF Aktiengesellschaft**
**Carl-Bosch-Strasse 38**
**W-6700 Ludwigshafen(DE)**

(72) Erfinder: **Seele, Rainer, Dr.**
**Leiblstrasse 3**
**W-6701 Fussgoenheim(DE)**
Erfinder: **Goetz, Norbert, Dr.**
**Schoefferstrasse 25**
**W-6520 Worms 1(DE)**
Erfinder: **Saupe, Thomas, Dr.**
**Kressenwiesenweg 13**
**W-6902 Sandhausen(DE)**
Erfinder: **Lorenz, Gisela, Dr.**
**Erlenweg 13**
**W-6730 Neustadt(DE)**
Erfinder: **Ammermann, Eberhard, Dr.**
**Sachsenstrasse 3**
**W-6700 Ludwigshafen(DE)**

(54) **1-Halogen-1-azolylmethanderivate und diese enthaltende Fungizide.**

(57) 1-Halogen-1-azolylmethanderivate der allgemeinen Formel

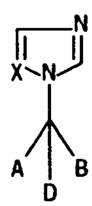

in welcher

A und B Alkyl, Benzyl, Benzoyl, Acyl, Biphenylyl, Naphthyl, Heteroaryl oder Phenyl bedeuten, wobei jeder dieser Reste substituiert sein kann;

D den Rest Cl oder Br bedeutet,

X den Rest CH oder N bedeutet,

sowie ihre pflanzenverträglichen Säureadditionssalze und Metallkomplexverbindungen und diese Verbindungen enthaltende Fungizide.

EP 0 483 615 A2

Die vorliegende Erfindung betrifft neue 1-Halogen-1-azolylmethanderivate, diese enthaltende Fungizide, Verfahren zu ihrer Herstellung und ihre Anwendung als Fungizide. Die neuen Verbindungen haben eine gute fungizide Wirkung.

Es ist bekannt, 1-Halogen-1-azolylethanderivate als Fungizide zu verwenden (EP 346 727). Ihre Wirkung ist jedoch unbefriedigend.

Es wurde nun gefunden, daß 1-Halogen-1-azolylmethanderivate der allgemeinen Formel I

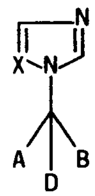

in welcher

A und B gleich oder verschieden sind und $C_1$- bis $C_8$-Alkyl, $C_3$- bis $C_8$-Cycloalkyl, Benzyl, Benzoyl, $C_1$- bis $C_8$-Acyl, Biphenylyl, Naphthyl, Heteroaryl oder Phenyl bedeuten, wobei jeder dieser Reste einfach bis dreifach durch Halogen, Nitro, Phenoxy, Amino, $C_1$- bis $C_4$-Alkyl, $C_1$- bis $C_4$-Alkoxy oder $C_1$- bis $C_4$-Halogenalkylsubstituiert sein kann;

D den Rest Cl oder Br bedeutet,

X den Rest CH oder N bedeutet,

sowie ihre pflanzenverträglichen Säureadditionssalze oder Metallkomplexe eine bessere fungizide Wirkung besitzen als bekannte Azolverbindungen.

Im einzelnen haben die Substituenten in Formel I beispielsweise folgende Bedeutung:

A und B unabhängig voneinander

unverzweigtes oder verzweigtes $C_1$-$C_8$-Alkyl, insbesondere $C_1$-$C_4$-Alkyl wie Methyl, Ethyl, Propyl, Isopropyl, Butyl, iso-Butyl, sec.-Butyl, tert.-Butyl,

$C_3$-$C_8$-Cycloalkyl wie Cyclopropyl, Cyclopentyl, Cyclohexyl, Benzyl und halogensubstituiertes Benzyl wie 2-Chlorbenzyl, 3-Chlorbenzyl, 4-Chlorbenzyl, 2-Fluorbenzyl, 3-Fluorbenzyl, 4-Fluorbenzyl sowie 2-Brombenzyl, 3-Brombenzyl und 4-Brombenzyl, 2,3-Dichlorbenzyl, 2,4-Dichlorbenzyl, 2,5-Dichlorbenzyl und 2,6-Dichlorbenzyl;

einfach durch Nitro-, Phenoxy-, Amino- oder $C_1$- bis $C_4$-Alkylgruppen substituiertes Benzyl, wie 4-Nitrobenzyl, 4-Phenoxybenzyl, 4-Aminobenzyl sowie 4-Methylbenzyl, 4-Isopropylbenzyl und 4-tert.-Butylbenzyl;

einfach oder zweifach durch $C_1$- bis $C_4$-Alkoxygruppen substituiertes Benzyl, wie 2-Methoxybenzyl, 3-Methoxybenzyl, 4-Methoxybenzyl, 4-tert.-Butyloxybenzyl und 2,4-Dimethoxybenzyl;

dreifach halogensubstituiertes Methylbenzyl, wie 2-Trifluormethylbenzyl, 3-Trifluormethylbenzyl und 4-Trifluormethylbenzyl;

Benzoyl und halogensubstituiertes Benzoyl, wie 2-Chlorbenzoyl, 3-Chlorbenzoyl, 4-Chlorbenzoyl, 2-Fluorbenzoyl, 4-Fluorbenzoyl sowie 2-Brombenzoyl, 3-Brombenzoyl und 4-Brombenzoyl, 2,3-Dichlorbenzoyl, 2,4-Dichlorbenzoyl und 2,5-Dichlorbenzoyl;

einfach durch Nitro-, Phenoxy-, Amino- oder $C_1$- bis $C_4$-Alkylgruppen substituiertes Benzoyl, wie 4-Nitrobenzoyl, 4-Phenoxybenzoyl, 4-Aminobenzoyl und 4-Methylbenzoyl;

einfach oder zweifach durch $C_1$- bis $C_4$-Alkoxygruppen substituiertes Benzoyl, wie 2-Methoxybenzoyl, 3-Methoxybenzoyl, 4-Methoxybenzoyl und 2,4-Dimethoxybenzoyl;

dreifach halogensubstituiertes Methylbenzoyl, wie 2-Trifluormethylbenzoyl, 3-Trifluormethylbenzoyl und 4-Trifluormethylbenzoyl;

Naphthyl wie 1-Naphthyl und 2-Naphthyl;

Biphenylyl wie o-, m- oder p-Biphenylyl;

Phenyl und halogensubstituiertes Phenyl, wie 2-Chlorphenyl, 3-Chlorphenyl, 4-Chlorphenyl, 2-Fluorphenyl, 3-Fluorphenyl, 4-Fluorphenyl sowie 2-Bromphenyl, 3-Bromphenyl und 4-Bromphenyl, 2,3-Dichlorphenyl, 2,4-Dichlorphenyl, 2,5-Dichlorphenyl und 2,6-Dichlorphenyl;

einfach durch Nitro-, Phenoxy-, Amino- oder $C_1$- bis $C_4$-Alkylgruppen substituiertes Phenyl, wie 3-Nitrophenyl, 4-Nitrophenyl, 3-Phenoxyphenyl, 4-Phenoxyphenyl, 3-Aminophenyl und 4-Aminophenyl sowie 4-Methylphenyl, 4-Isopropylphenyl und 4-tert.-Butylphenyl;

einfach oder zweifach durch $C_1$- bis $C_4$-Alkoxygruppen substituiertes Phenyl, wie 2-Methoxyphenyl, 3-Methoxyphenyl, 4-Methoxyphenyl, 4-tert.-Butyloxyphenyl und 2,3-Dimethoxyphenyl sowie 2,4-Dimethoxyphenyl;
dreifach halogensubstituiertes Methylphenyl, wie 2-Trifluormethyl-, 3-Trifluormethyl- und 4-Trifluormethylphenyl;
5- oder 6-gliedriges Heteroaryl, wie Pyrrolyl, Pyrrol-2-yl, Pyrrol-3-yl, Furanyl, Furan-2-yl, Thien-2-yl, Thien-3-yl, Pyrid-2-yl, Pyrid-3-yl, Pyrid-4-yl, 1,3-Diazol-2-yl, Oxazol-4-yl, Oxazol-5-yl, Isoxazolyl, Isoxazol-3-yl, Isoxazol-4-yl, Isoxazol-5-yl, Thiazol-4-yl, Thiazol-5-yl;
$C_1$-$C_8$-Acyl, wie $C_1$-$C_4$-Alkylcarbonyl, wie Acetyl oder Propionyl.

Die Verbindungen der Formel I enthalten asymmetrische C-Atome und können daher als Enantiomere und Diastereomere auftreten. Die Erfindung umfaßt sowohl die reinen Isomere als auch deren Gemische. Die Gemische von Diastereomeren können nach bekannten Methoden, zum Beispiel durch fraktionierte Kristallisation oder durch Chromatographie an Kieselgel in die Komponenten getrennt werden. Die Racemate lassen sich bei den erfindungsgemäßen Verbindungen mit üblichen Methoden, beispielsweise durch Salzbildung mit einer optisch aktiven Säure, Trennung der diastereomeren Salze und Freisetzung der Enantiomeren mittels einer Base, trennen.

Als fungizide Wirkstoffe können sowohl die einzelnen Diastereomere bzw. Enantiomere als auch deren Gemische verwendet werden.

Säureadditionssalze sind beispielsweise die Hydrochloride, Bromide, Sulfate, Nitrate, Phosphate, Oxalate, oder Dodecylbenzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der 1-Halogen-1-azolylmethanderivate mit Säuren.

Metallkomplexe der Wirkstoffe I oder ihrer Salze können z.B. mit Kupfer, Zink, Zinn, Mangan, Eisen, Kobalt oder Nickel gebildet werden, indem man die 1-Halogen-1-azolylmethanderivate mit entsprechenden Metallsalzen umsetzt.

Die erfindungsgemäßen Verbindungen I stellt man auf sehr vorteilhafte Weise analog der von H. Matsumoto et al (Tetrahedron Letters. 52, 5011 (1979)) beschriebenen Methode durch Umsetzung eines Ketons der Formel II mit einem Azol der Formel III und einem Säurehalogenid ($YD_2$) entsprechend folgender Summengleichung her:

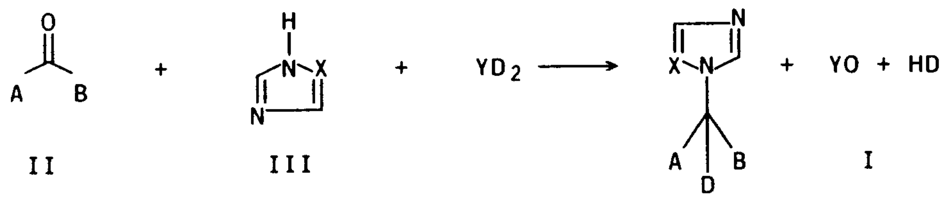

II III I

Die anorganischen Säurehalogenide ($YD_2$) sind Halogenierungsmittel wie Phosphoroxidchlorid, Thiophosgen, bevorzugt Phosgen, Thionylchlorid und -bromid.

Das Säurehalogenid wird bevorzugt in mindestens äquimolaren Mengen bezogen auf das Keton II eingesetzt. Die Azolkomponente III wird z.B. in der mehr als zweifachen, bevorzugt in der 5-6fachen molaren Menge, bezogen auf das Säurechlorid bzw. -bromid, eingesetzt.

Die Umsetzung erfolgt bevorzugt bei Temperaturen zwischen -30 und +100°C, besonders bevorzugt zwischen 0 und 20°C in Gegenwart eines Lösungsmittels.

Bevorzugte Lösungsmittel sind z.B. Nitrile wie Acetonitril, Ether wie Tetrahydrofuran, Diethylether oder Dioxan. Besonders bevorzugt werden Kohlenwasserstoffe und Chlorkohlenwasserstoffe wie Hexan, Benzol, Toluol, Methylenchlorid, Tetrachlorkohlenstoff oder Gemische der genannten Solventien.

Im allgemeinen arbeitet man bei Atmosphärendruck, sofern sich nicht wegen leichtflüchtiger Reaktionspartner ein höherer Druck, etwa bis zu 5 bar empfiehlt.

Da die Säurehalogenide und die intermediär entstehenden Zwischenprodukte hydrolyseempfindlich sind, arbeitet man bevorzugt unter Feuchtigkeitsausschluß, besonders bevorzugt in einer Schutzgasatmosphäre.

Das nachfolgende Beispiel erläutert die Herstellung der Wirkstoffe.

Herstellungsbeispiel

Beispiel 1

1,3-Diphenyl-2-chlor-2-(1,2,4-triazol-1-yl)-propan

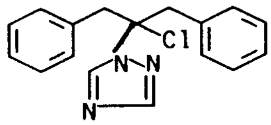

Eine Lösung aus 98,6 g (1,43 mol) Triazol in 250 ml Methylenchlorid wurde bei 0°C unter Stickstoffatmosphäre mit 42,7 g (0,39 mol) Thionylchlorid und nach 30 minütigem Rühren bei 25°C mit 50 g (0,24 mol) Diphenylaceton versetzt.

Nach 12-stündiger Reaktionszeit bei 25°C wurden 200 ml Wasser zugegeben, wonach die abgetrennte wäßrige Phase zweimal mit Methylenchlorid extrahiert wurde. Die vereinigten organischen Phasen wurden sodann wie üblich auf das Triazolderivat aufgearbeitet.

Ausbeute: 56,4 g (79 %)
Schmp.: 100 bis 102°C

Analog Beispiel 1 können die in der Tabelle aufgeführten Verbindungen hergestellt werden.

Tabelle

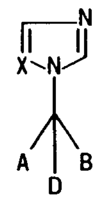

| Bsp. | A | B | D | X | Schmp./IR |
|---|---|---|---|---|---|
| 1 | $CH_2C_6H_5$ | $-CH_2C_6H_5$ | Cl | N | 100-102°C |
| 2 | $CH_2C_6H_5$ | $CH_3$ | Cl | N | |
| 3 | $CH_2C_6H_5$ | $C_3H_7$ | Cl | N | |
| 4 | $CH_2C_6H_5$ | Cyclohexyl | Cl | N | |
| 5 | $CH_2C_6H_5$ | 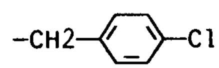 | Cl | N | |
| 6 | $CH_2C_6H_5$ | 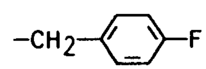 | Cl | N | |
| 7 | $CH_2C_6H_5$ | 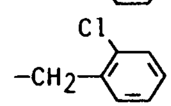 | Cl | N | |
| 8 | $CH_2C_6H_5$ | 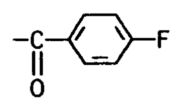 | Cl | N | |
| 9 | $CH_2C_6H_5$ | 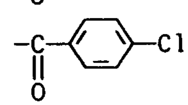 | Cl | N | |

| Bsp. | A | B | D | X | Schmp./IR |
|---|---|---|---|---|---|
| 10 | $CH_2C_6H_5$ | $-CO-CH_3$ | Cl | N | |
| 11 | $CH_2C_6H_5$ | $-CO-C_3H_7$ | Cl | N | |
| 12 | $CH_2C_6H_5$ | 4-Biphenylyl | Cl | N | |
| 13 | $CH_2C_6H_5$ | 1-Naphthyl | Cl | N | |
| 14 | $CH_2C_6H_5$ | $C_6H_5$ | Cl | N | |
| 15 | $CH_2C_6H_5$ | $2-Cl-C_6H_4$ | Cl | N | |
| 16 | $CH_2C_6H_5$ | $4-Cl-C_6H_4$ | Cl | N | |
| 17 | $CH_2C_6H_5$ | $2,4-Cl_2-C_6H_3$ | Cl | N | |
| 18 | $CH_2C_6H_5$ | $2-F-C_6H_4$ | Cl | N | |
| 19 | $CH_2C_6H_5$ | $4-F-C_6H_4$ | Cl | N | |
| 20 | $CH_2C_6H_5$ | $4-CH_3-C_6H_4$ | Cl | N | |
| 21 | $CH_2C_6H_5$ | $4-OCH_3-C_6H_4$ | Cl | N | |
| 22 | $CH_2C_6H_5$ | $2-CF_3-C_6H_4$ | Cl | N | |
| 23 | 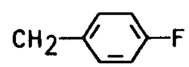 | $CH_3$ | Cl | N | |
| 24 | 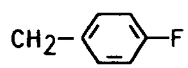 | $C_3H_7$ | Cl | N | |
| 25 | 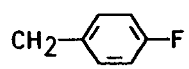 | $C_4H_9$ | Cl | N | |
| 26 | 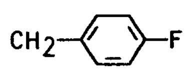 | Cyclohexyl | Cl | N | |
| 27 | 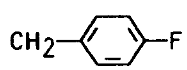 | $-CH_2-C_6H_5$ | Cl | N | |
| 28 | 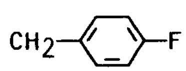 | 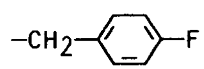 | Cl | N | 150°C |

| Bsp. | A | B | D | X | Schmp./IR |
|---|---|---|---|---|---|
| 29 | 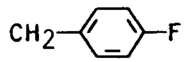 | 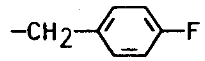 | Cl | CH | |
| 30 | 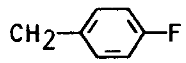 | 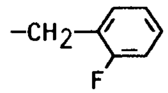 | Cl | N | Harz |
| 31 | 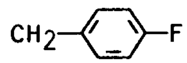 | 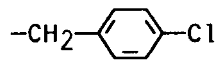 | Cl | N | |
| 32 | 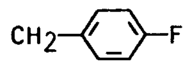 | 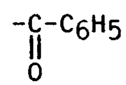 | Cl | N | |
| 33 | 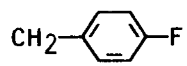 | 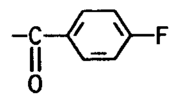 | Cl | N | |
| 34 | 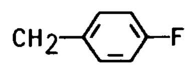 | 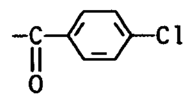 | Cl | N | |
| 35 | 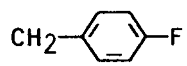 | $-CO-CH_3$ | Cl | N | |
| 36 | 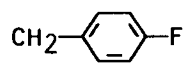 | $-CO-C_3H_7$ | Cl | N | |
| 37 | 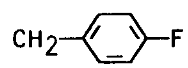 | 4-Biphenylyl | Cl | N | |
| 38 | 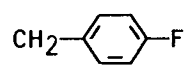 | 1-Naphthyl | Cl | N | |
| 39 | 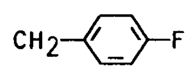 | $C_6H_5$ | Cl | N | |
| 40 | 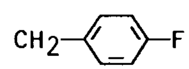 | $2-Cl-C_6H_4$ | Cl | N | |
| 41 | 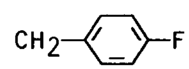 | $4-Cl-C_6H_4$ | Cl | N | |

| Bsp. | A | B | D | X | Schmp./IR |
|---|---|---|---|---|---|
| 42 | 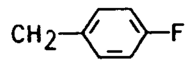 | 2,4-$Cl_2$-$C_6H_3$ | Cl | N | |
| 43 | 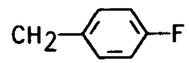 | 2-F-$C_6H_4$ | Cl | N | |
| 44 | 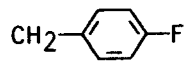 | 4-F-$C_6H_4$ | Cl | N | |
| 45 | 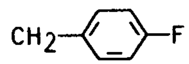 | 4-$CH_3$-$C_6H_4$ | Cl | N | |
| 46 | 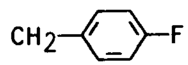 | 4-$OCH_3$-$C_6H_4$ | Cl | N | |
| 47 | 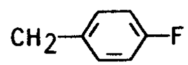 | 4-$CF_3$-$C_6H_4$ | Cl | N | |
| 48 | 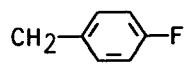 | 2-Furyl | Cl | N | |
| 49 | 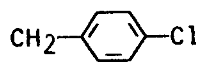 | $CH_3$ | Cl | N | |
| 50 | 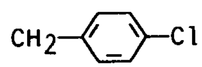 | $C_3H_7$ | Cl | N | |
| 51 | 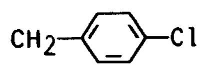 | 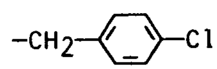 | Cl | N | |
| 52 | 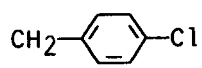 | 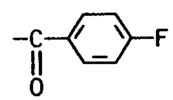 | Cl | N | |
| 53 | 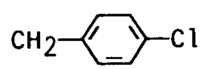 | 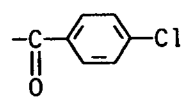 | Cl | N | |
| 54 | 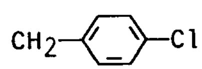 | -CO-$CH_3$ | Cl | N | |
| 55 | 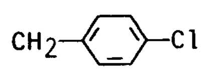 | -CO-$C_3H_7$ | Cl | N | |

| Bsp. | A | B | D | X | Schmp./IR |
|---|---|---|---|---|---|
| 56 | 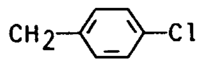 | $C_6H_5$ | Cl | N | |
| 57 | 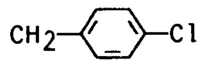 | $4-Cl-C_6H_4$ | Cl | N | |
| 58 | 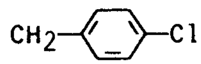 | $4-F-C_6H_4$ | Cl | N | |
| 59 | 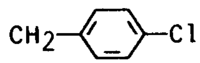 | $2,4-Cl_2-C_6H_3$ | Cl | N | |
| 60 | 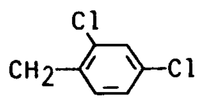 | $CH_3$ | Cl | N | |
| 61 | 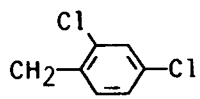 | $C_4H_9$ | Cl | N | 2960, 1588, 1475, 1280 $cm^{-1}$ |
| 62 | 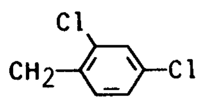 | $CH_2-C_6H_5$ | Cl | N | |
| 63 | 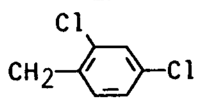 | 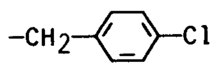 | Cl | N | |
| 64 | 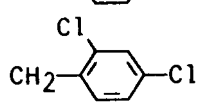 | 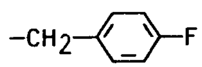 | Cl | N | |
| 65 | 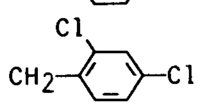 | 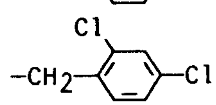 | Cl | N | |
| 66 | 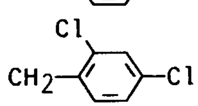 | 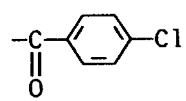 | Cl | N | |

| Bsp. | A | B | D | X | Schmp./IR |
|---|---|---|---|---|---|
| 67 | 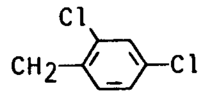 | 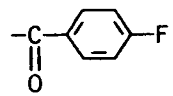 | Cl | N | |
| 68 | 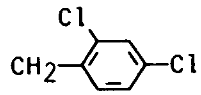 | $-CO-CH_3$ | Cl | N | |
| 69 | 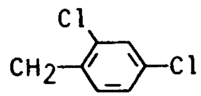 | $-CO-C_4H_9$ | Cl | N | |
| 70 | 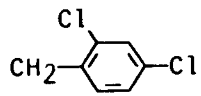 | $C_6H_5$ | Cl | N | |
| 71 | 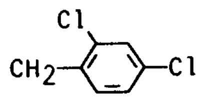 | $4-Cl-C_6H_4$ | Cl | N | |
| 72 | 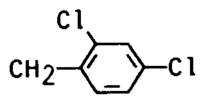 | $2-Cl-C_6H_4$ | Cl | N | |
| 73 | 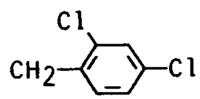 | $2,4-Cl_2-C_6H_3$ | Cl | N | |
| 74 | 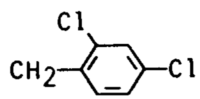 | $4-F-C_6H_4$ | Cl | N | |
| 75 | 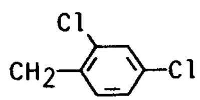 | $4-CH_3-C_6H_4$ | Cl | N | |
| 76 | 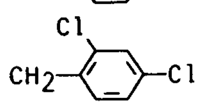 | $4-CF_3-C_6H_4$ | Cl | N | |
| 77 | 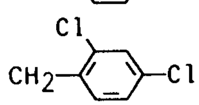 | Cyclohexyl | Cl | N | |

| Bsp. | A | B | D | X | Schmp./IR |
|---|---|---|---|---|---|
| 78 | $-\underset{\overset{\|}{O}}{C}-C_6H_5$ | $CH_3$ | Cl | N | |
| 79 | $-\underset{\overset{\|}{O}}{C}-C_6H_5$ | $C_3H_7$ | Cl | N | |
| 80 | $-\underset{\overset{\|}{O}}{C}-C_6H_5$ | $C_4H_9$ | Cl | N | |
| 81 | $-\underset{\overset{\|}{O}}{C}-C_6H_5$ | Cyclohexyl | Cl | N | |
| 82 | $-\underset{\overset{\|}{O}}{C}-C_6H_5$ | $C_6H_5$ | Cl | N | 1698, 1502, 1447, 1231, 743 $cm^{-1}$ |
| 83 | $-\underset{\overset{\|}{O}}{C}-C_6H_5$ | $2-Cl-C_6H_4$ | Cl | N | |
| 84 | $-\underset{\overset{\|}{O}}{C}-C_6H_5$ | $4-Cl-C_6H_4$ | Cl | N | |
| 85 | $-\underset{\overset{\|}{O}}{C}-C_6H_5$ | $2,4-Cl_2-C_6H_3$ | Cl | N | |
| 86 | $-\underset{\overset{\|}{O}}{C}-C_6H_5$ | $2-F-C_6H_4$ | Cl | N | |
| 87 | $-\underset{\overset{\|}{O}}{C}-C_6H_5$ | $4-F-C_6H_4$ | Cl | N | |
| 88 | $-\underset{\overset{\|}{O}}{C}-C_6H_5$ | $2-Br-C_6H_4$ | Cl | N | |

| Bsp. | A | B | D | X | Schmp./IR |
|---|---|---|---|---|---|
| 89 | 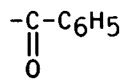 | $2-CH_3-C_6H_4$ | Cl | N | |
| 90 | 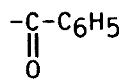 | $4-CH_3-C_6H_4$ | Cl | N | |
| 91 | 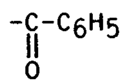 | $2-CF_3-C_6H_4$ | Cl | N | |
| 92 | 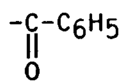 | $4-CF_3-C_6H_4$ | Cl | N | |
| 93 | 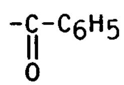 | $4-OCH_3-C_6H_4$ | Cl | N | |
| 94 | 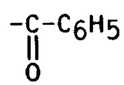 | 1-Naphthyl | Cl | N | |
| 95 | 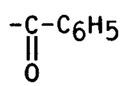 | 2-Naphthyl | Cl | N | |
| 96 | 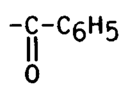 | 4-Biphenyl | Cl | N | |
| 97 | 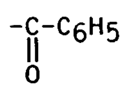 | 2-Thienyl | Cl | N | |
| 98 | 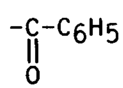 | 3-Thienyl | Cl | N | |
| 99 | 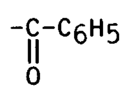 | 2-Pyridyl | Cl | N | |

| Bsp. | A | B | D | X | Schmp./IR |
|---|---|---|---|---|---|
| 100 | 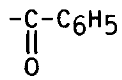 | 3-Pyridyl | Cl | N | |
| 101 | 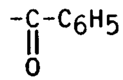 | 4-Pyridyl | Cl | N | |
| 102 | 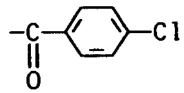 | $CH_3$ | Cl | N | |
| 103 | 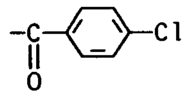 | $C_3H_7$ | Cl | N | |
| 104 | 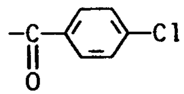 | $C_4H_9$ | Cl | N | |
| 105 | 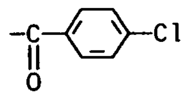 | Cyclohexyl | Cl | N | |
| 106 | 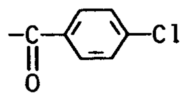 | $C_6H_5$ | Cl | N | |
| 107 | 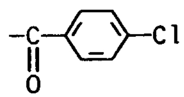 | $2\text{-}Cl\text{-}C_6H_4$ | Cl | N | |
| 108 | 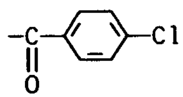 | $4\text{-}Cl\text{-}C_6H_4$ | Cl | N | Harz |
| 109 | 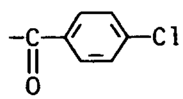 | $2,4\text{-}Cl_2\text{-}C_6H_3$ | Cl | N | |
| 110 | 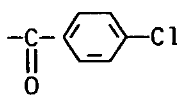 | $2\text{-}F\text{-}C_6H_4$ | Cl | N | |

| Bsp. | A | B | D | X | Schmp./IR |
|---|---|---|---|---|---|
| 111 | 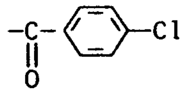 | 4-F-$C_6H_4$ | Cl | N | |
| 112 | 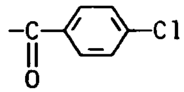 | 4-$CH_3$-$C_6H_4$ | Cl | N | |
| 113 | 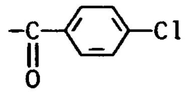 | 4-$CF_3$-$C_6H_4$ | Cl | N | |
| 114 | 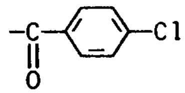 | 3-Pyridyl | Cl | N | |
| 115 | 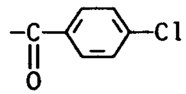 | 2-Furyl | Cl | N | |
| 116 | 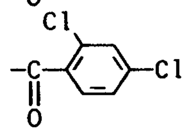 | $CH_3$ | Cl | N | |
| 117 | 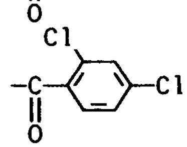 | Cyclohexyl | Cl | N | |
| 118 | 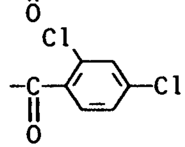 | $C_6H_5$ | Cl | N | |
| 119 | 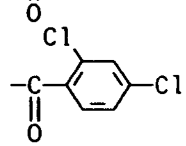 | 2-Cl-$C_6H_4$ | Cl | N | |
| 120 | 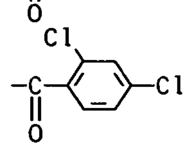 | 4-Cl-$C_6H_4$ | Cl | N | |

| Bsp. | A | B | D | X | Schmp./IR |
|---|---|---|---|---|---|
| 121 | 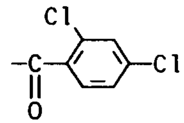 | 2,4-$Cl_2$-$C_6H_3$ | Cl | N | |
| 122 | 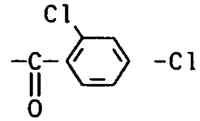 | 2-F-$C_6H_4$ | Cl | N | |
| 123 | 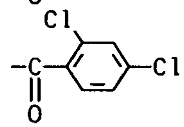 | 4-F-$C_6H_4$ | Cl | N | |
| 124 | 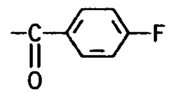 | $CH_3$ | Cl | N | |
| 125 | 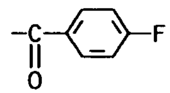 | $C_3H_7$ | Cl | N | |
| 126 | 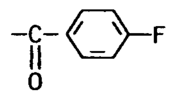 | $C_4H_9$ | Cl | N | |
| 127 | 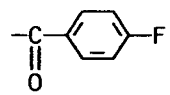 | Cyclohexyl | Cl | N | |
| 128 | 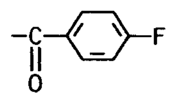 | $C_6H_5$ | Cl | N | |
| 129 | 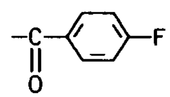 | 2-Cl-$C_6H_4$ | Cl | N | |
| 130 | 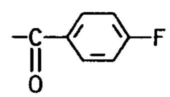 | 4-Cl-$C_6H_4$ | Cl | N | |

| Bsp. | A | B | D | X | Schmp./IR |
|---|---|---|---|---|---|
| 131 | 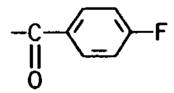 | 2,4-$Cl_2$-$C_6H_3$ | Cl | N | |
| 132 | 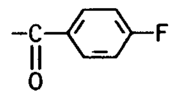 | 2-F-$C_6H_4$ | Cl | N | |
| 133 | 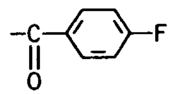 | 4-F-$C_6H_4$ | Cl | N | 1701, 1598, 1508, 1237, 1161 $cm^{-1}$ |
| 134 | 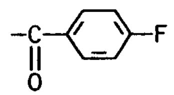 | 2-Br-$C_6H_4$ | Cl | N | |
| 135 | 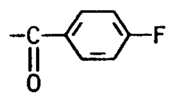 | 4-Br-$C_6H_4$ | Cl | N | |
| 136 | 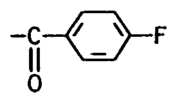 | 2-$CH_3$-$C_6H_4$ | Cl | N | |
| 137 | 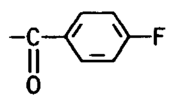 | 4-$CH_3$-$C_6H_4$ | Cl | N | |
| 138 | 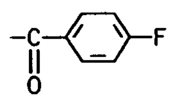 | 2-$CF_3$-$C_6H_4$ | Cl | N | |
| 139 | 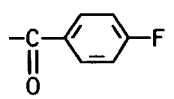 | 4-$CF_3$-$C_6H_4$ | Cl | N | |
| 140 | 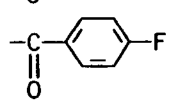 | 1-Naphthyl | Cl | N | |
| 141 | 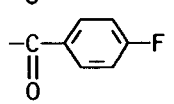 | 2-Naphthyl | Cl | N | |

| Bsp. | A | B | D | X | Schmp./IR |
|---|---|---|---|---|---|
| 142 | 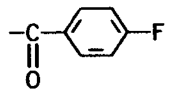 | 4-Biphenylyl | Cl | N | |
| 143 | 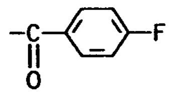 | 2-Furyl | Cl | N | |
| 144 | 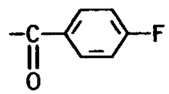 | 2-Thienyl | Cl | N | |
| 145 | 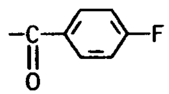 | 3-Pyridyl | Cl | N | |
| 146 | 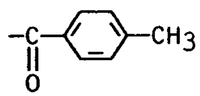 | $CH_3$ | Cl | N | |
| 147 | 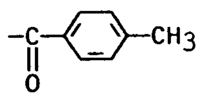 | Cyclohexyl | Cl | N | |
| 148 | 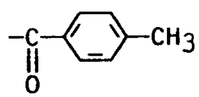 | $C_6H_5$ | Cl | N | |
| 149 | 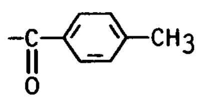 | $2-Cl-C_6H_4$ | Cl | N | |
| 150 | 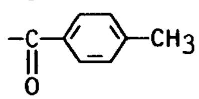 | $4-Cl-C_6H_4$ | Cl | N | |
| 151 | 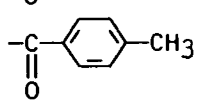 | $2,4-Cl_2-C_6H_3$ | Cl | N | |
| 152 | 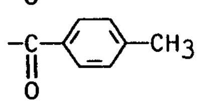 | $2-F-C_6H_4$ | Cl | N | |

| Bsp. | A | B | D | X | Schmp./IR |
|---|---|---|---|---|---|
| 153 | 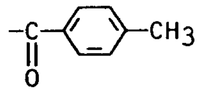 | 4-F-$C_6H_4$ | Cl | N | |
| 154 | 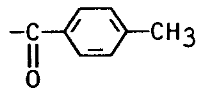 | 4-$CH_3$-$C_6H_4$ | Cl | N | |
| 155 | 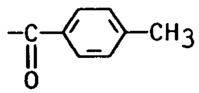 | 4-$CF_3$-$C_6H_4$ | Cl | N | |
| 156 | 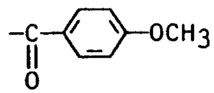 | $CH_3$ | Cl | N | |
| 157 | 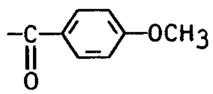 | Cyclohexyl | Cl | N | |
| 158 | 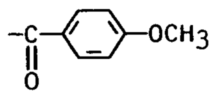 | 4-Cl-$C_6H_4$ | Cl | N | |
| 159 | 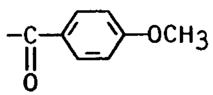 | 4-F-$C_6H_4$ | Cl | N | |
| 160 | 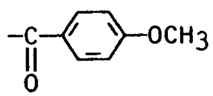 | 4-$OCH_3$-$C_6H_4$ | Cl | N | |
| 161 | 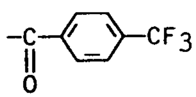 | $CH_3$ | Cl | N | |
| 162 | 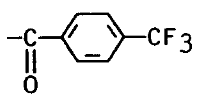 | $C_3H_7$ | Cl | N | |
| 163 | 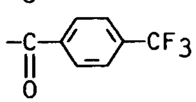 | Cyclohexyl | Cl | N | |

| Bsp. | A | B | D | X | Schmp./IR |
|---|---|---|---|---|---|
| 164 | 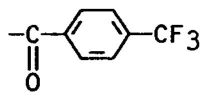 | $2-Cl-C_6H_4$ | Cl | N | |
| 165 | 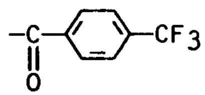 | $4-Cl-C_6H_4$ | Cl | N | |
| 166 | 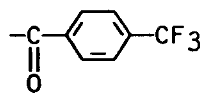 | $2,4-Cl_2-C_6H_3$ | Cl | N | |
| 167 | 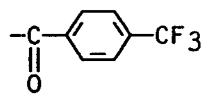 | $4-F-C_6H_4$ | Cl | N | |
| 168 | 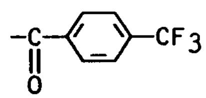 | $4-CH_3-C_6H_4$ | Cl | N | |
| 169 | 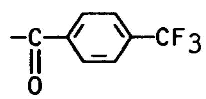 | $4-CF_3-C_6H_4$ | Cl | N | |
| 170 | 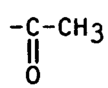 | $CH_3$ | Cl | N | |
| 171 | 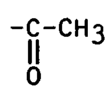 | Cyclohexyl | Cl | N | |
| 172 | 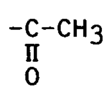 | $C_6H_5$ | Cl | N | 105-107°C |
| 173 | 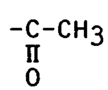 | $2-Cl-C_6H_4$ | Cl | N | |

| Bsp. | A | B | D | X | Schmp./IR |
|---|---|---|---|---|---|
| 174 | $-\underset{\underset{O}{\|}}{C}-CH_3$ | $4-Cl-C_6H_4$ | Cl | N | |
| 175 | $-\underset{\underset{O}{\|}}{C}-CH_3$ | $2,4-Cl_2-C_6H_3$ | Cl | N | |
| 176 | $-\underset{\underset{O}{\|}}{C}-CH_3$ | $2-F-C_6H_4$ | Cl | N | |
| 177 | $-\underset{\underset{O}{\|}}{C}-CH_3$ | $4-F-C_6H_4$ | Cl | N | |
| 178 | $-\underset{\underset{O}{\|}}{C}-CH_3$ | $4-CH_3-C_6H_4$ | Cl | N | |
| 179 | $-\underset{\underset{O}{\|}}{C}-CH_3$ | $4-CF_3-C_6H_4$ | Cl | N | |
| 180 | $-\underset{\underset{O}{\|}}{C}-CH_3$ | 3-Pyridyl | Cl | N | |
| 181 | $-\underset{\underset{O}{\|}}{C}-CH_3$ | 2-Thienyl | Cl | N | |
| 182 | $-\underset{\underset{O}{\|}}{C}-C_3H_7$ | $CH_3$ | Cl | N | |
| 183 | $-\underset{\underset{O}{\|}}{C}-C_3H_7$ | Cyclohexyl | Cl | N | |
| 184 | $-\underset{\underset{O}{\|}}{C}-C_3H_7$ | $C_6H_5$ | Cl | N | |

| Bsp. | A | B | D | X | Schmp./IR |
|---|---|---|---|---|---|
| 185 | $-C(=O)-C_3H_7$ | $2-Cl-C_6H_4$ | Cl | N | |
| 186 | $-C(=O)-C_3H_7$ | $4-Cl-C_6H_4$ | Cl | N | |
| 187 | $-C(=O)-C_3H_7$ | $2,4-Cl_2-C_6H_3$ | Cl | N | |
| 188 | $-C(=O)-C_3H_7$ | $2-F-C_6H_4$ | Cl | N | |
| 189 | $-C(=O)-C_3H_7$ | $4-F-C_6H_4$ | Cl | N | |
| 190 | $-C(=O)-C_3H_7$ | $4-CH_3-C_6H_4$ | Cl | N | |
| 191 | $-C(=O)-C_3H_7$ | $4-CF_3-C_6H_4$ | Cl | N | |
| 192 | Cyclohexyl | $CH_3$ | Cl | N | |
| 193 | Cyclohexyl | $C_3H_7$ | Cl | N | |
| 194 | Cyclohexyl | $2-Cl-C_6H_4$ | Cl | N | |
| 195 | Cyclohexyl | $4-Cl-C_6H_4$ | Cl | N | |
| 196 | Cyclohexyl | $4-F-C_6H_4$ | Cl | N | |
| 197 | Cyclohexyl | $4-CH_3-C_6H_4$ | Cl | N | |
| 198 | Cyclohexyl | Cyclohexyl | Cl | N | |

Die neuen Verbindungen eignen sich als Fungizide.

Die fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin), Dimethylformamid und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen der Verbindung Nr. 1 und 10 Gew.-Teilen N-Methyl-$\alpha$-pyrrolidon, die zur Anwendung in Form kleinster Tropfen geeignet ist;

II. eine Mischung aus 20 Gew.-Teilen der Verbindung Nr. 28, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 61, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 82, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 133, 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 1 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 28, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 61, 10 Gew.-Teilen des Natriumsalzes eines Phenolsulfonsäureharnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 82, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Anwendungsbeispiele

Als bekannte Vergleichsverbindung wurde 1-Chlor-1-(1,2,4-triazol-1-yl)-2-phenyl-3-(4-fluorphenyl)-propan (A) - bekannt aus EP 346 727 - verwendet.

Anwendungsbeispiel 1

Wirksamkeit gegen Weizenmehltau

Blätter von in Töpfen gewachsenen Weizenkeimlingen der Sorte "Kanzler" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe

graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Das Ergebnis des Versuches zeigt, daß die Wirkstoffe Nr. 1, 28, 30 und 61 bei der Anwendung als 250 ppm Wirkstoff enthaltende wäßrige Dispersion eine bessere fungizide Wirkung zeigen (99 %) als die bekannte Vergleichsverbindung A (75 %).

Anwendungsbeispiel 2

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Das Ergebnis des Versuches zeigt, daß die Wirkstoffe Nr. 28 und 30 bei der Anwendung als 250 ppm Wirkstoff enthaltende wäßrige Dispersion eine bessere fungizide Wirkung zeigen (93 %) als die bekannte Vergleichsverbindung A (50 %).

**Patentansprüche**

**1.** 1-Halogen-1-azolylmethanderivate der allgemeinen Formel I

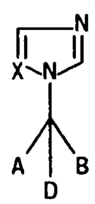

I

in welcher

A und B gleich oder verschieden sind und $C_1$- bis $C_8$-Alkyl, $C_3$- bis $C_8$-Cycloalkyl, Benzyl, Benzoyl, $C_1$- bis $C_8$-Acyl, Biphenylyl, Naphthyl, Heteroaryl oder Phenyl bedeuten, wobei jeder dieser Reste einfach bis dreifach durch Halogen, Nitro, Phenoxy, Amino, $C_1$- bis $C_4$-Alkyl, $C_1$- bis $C_4$-Alkoxy oder $C_1$- bis $C_4$-Halogenalkyl substituiert sein kann;

D den Rest Cl oder Br bedeutet,

X den Rest CH oder N bedeutet,

sowie ihre pflanzenverträglichen Säureadditionssalze und Metallkomplexverbindungen.

**2.** Verfahren zur Herstellung der 1-Halogen-1-azolylmethanderivate der Formel I gemäß Anspruch 1, dadurch gekennzeichnet, daß man ein Keton der Formel II

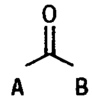

II

in welcher A und B die in Anspruch 1 angegebenen Bedeutungen haben, mit einem Azol der Formel III

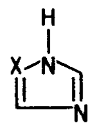

III

in Gegenwart eines anorganischen Säurechlorids oder -bromids umsetzt und die erhaltenen Verbindungen gewünschtenfalls in ihre Salze oder Metallkomplexe überführt.

3. Fungizides Mittel, enthaltend einen innerten Trägerstoff und eine fungizid wirksame Menge eines 1-Halogen-1-azolylmethanderivates der Formel I

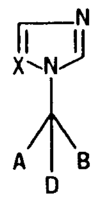

I

in welcher

A und B gleich oder verschieden sind und $C_1$- bis $C_8$-Alkyl, $C_3$- bis $C_8$-Cycloalkyl, Benzyl, Benzoyl, $C_1$- bis $C_8$-Acyl, Biphenylyl, Naphthyl, Heteroaryl oder Phenyl bedeutet, wobei jeder dieser Reste einfach bis dreifach durch Halogen, Nitro, Phenoxy, Amino, $C_1$- bis $C_4$-Alkyl, $C_1$- bis $C_4$-Alkoxy oder $C_1$- bis $C_4$-Halogenalkyl substituiert sein kann;

D den Rest Cl oder Br bedeutet,

X den Rest CH oder N bedeutet,

oder dessen pflanzenverträglichen Salzes oder Metallkomplexes.

4. Verfahren zur Bekämpfung von Pilzen, dadurch gekennzeichnet, daß man eine fungizid wirksame Menge eines 1-Halogen-1-azolylmethanderivates der Formel I

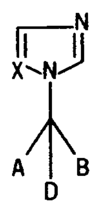

I

in welcher

A und B gleich oder verschieden sind und $C_1$- bis $C_8$-Alkyl, $C_3$- bis $C_8$-Cycloalkyl, Benzyl, Benzoyl, $C_1$- bis $C_8$-Acyl, Biphenylyl, Naphthyl, Heteroaryl oder Phenyl bedeuten, wobei jeder dieser Reste einfach bis dreifach durch Halogen, Nitro, Phenoxy, Amino, $C_1$- bis $C_4$-Alkyl, $C_1$- bis $C_4$-Alkoxy oder $C_1$- bis $C_4$-Halogenalkyl substituiert sein kann;

D den Rest Cl oder Br bedeutet;

X den Rest CH oder N bedeutet,

oder dessen pflanzenverträglichen Salzes oder Metallkomplexes auf Pilze oder vom Pilzbefall bedrohte Pflanzen, deren Lebensraum, Materialien, Holz oder Saatgut einwirken läßt.

5. Verbindung der Formel I gemäß Anspruch 1, in der A und B Benzyl, D Cl und X N bedeuten.

6. Verbindung der Formel I gemäß Anspruch 1, in der A und B 4-Fluorbenzyl, D Cl und X N bedeuten.

7. Verbindung der Formel I gemäß Anspruch 1, in der A 2,4-Dichlorbenzyl, B Butyl, D Cl und X N bedeuten.

**8.** Verbindung der Formel I gemäß Anspruch 1, in der A Benzoyl, B Phenyl, D Cl und X N bedeuten.

**9.** Verbindung der Formel 1 gemäß Anspruch 1, in der A 4-Fluorbenzoyl, B 4-Fluorphenyl, D Cl und X N bedeuten.